# EUROPEAN PATENT SPECIFICATION

(11) **EP 1 478 433 B1**
(45) Date of publication and mention of the grant of the patent: **04.11.2009**
(21) Application number: 03702212.6
(22) Date of filing: 28.02.2003
(51) Int. Cl.: A61N 1/05, H04R 25/00, A61N 1/36

(54) **CONNECTOR FOR DRUG DELIVERY SYSTEM IN COCHLEAR IMPLANT**
VERBINDUNGSELEMENT FÜR EIN MEDIKAMENTENABGABESYSTEM IN EINEM COCHLEAR-IMPLANTAT
RACCORD POUR SYSTEME DE DISTRIBUTION DE MEDICAMENT DANS UN IMPLANT COCHLEAIRE

(30) Priority: 28.02.2002 AU PS081502
(43) Date of publication of application: 24.11.2004
(73) Proprietor: Cochlear Limited, Lane Cove, NSW 2066 (AU)
(72) Inventor: Lenarz, Thomas Med. Hochschule Hannover, 30625 Hannover (DE); Paasche, Gerrit Med. Hochschule Hannover, 30625 Hannover (DE); Stoever, Timo Med. Hochschule Hannover, 30625 Hannover (DE)
(74) Representative: Schwan - Schwan - Schorer
(86) International application number: PCT/AU2003/000248
(87) International publication number: WO 2003/072193

(56) References cited:
- WO-A-00/71063
- WO-A1-02/32498
- WO-A1-02/41666
- US-A- 5 697 951
- US-A- 5 929 041
- US-B1- 6 309 410
- US-B1- 6 309 410
- US-B1- 6 421 569

## Description

### Field of the Invention

The present invention relates to a connector for use in association with a cochlear implant system.

### Background of the Invention

Hearing aids and cochlear implants have been proven to be useful in restoring the sensation of hearing to hearing impaired individuals.

In cases of mild hearing loss, wherein the essential structures of the cochlea are intact and the hair cells are able to detect the mechanical vibration of the cochlea fluid and transfer this into a neural impulse detected by the brain as sound, hearing aids are commonly used. Such hearing aids typically magnify the sound normally heard by the individual so that the individual experiences this sound sensation at a level at least approximately equivalent to that experienced by a normal hearing listener.

In cases where the hair cells of the cochlea have been damaged to the extent that they are no longer able to transfer the mechanical vibration of the cochlea fluid into an electrical signal, traditional hearing aids provide no benefit. In situations where there is such a severe or profound hearing loss, cochlear implants have been developed to restore the function of hearing to affected individuals. One such implant is described in United States Patent No. 4,532,930. The cochlear implant bypasses the role of the hair cells and directly delivers electrical stimulation to the nerves in the cochlea, representative of speech and environmental sounds, with the neural impulses generated by such electrical signals being sent to the brain and being interpreted as sound. The electrical stimulation is usually delivered to selected nerve sites within the cochlea by an array of electrodes that are electrically connected to an implanted stimulator device and mounted within a carrier member.

Traditionally, the implanted stimulator device receives a coded sound signal from an external sound processor device and from this coded signal the implanted stimulator directs the appropriate electrical stimulation to be provided to the appropriate electrode of the carrier member to reproduce the corresponding sound. The implanted stimulator is equipped with electronic circuitry and switches to allow stimulation to be delivered to a number of electrodes simultaneously or in very quick succession to provide detailed sound perception.

The external sound processor provides the coded signal to the implanted stimulator via a transcutaneous link, such as a radio frequency (RF) link, with the coded signal being directly representative of the surrounding sound as detected by an external microphone. The external microphone may be mounted on the external sound processor or may be remote from the external sound processor but connected via a suitable link.

With continuing technological advancements, it may be possible to provide a system which is totally implantable within the head of the user and having an internal microphone capable of detecting the external environmental sounds. In such a system the sound processor would also be implanted within the head of the user and process the sound signal in much the same way as the previously described system.

In such a system, the basic function of the sound processor is to take an audio signal from a microphone and then process it according to a particular speech coding strategy to produce a signal that contains stimulation information for the implant. In earlier speech coding strategies, the processor attempted to identify the important sounds present in the signal (such as speech) and encode them as patterns of electrical stimulation. In more recent strategies, the full range of spectral and temporal information in the audio signal has been provided to the user without any attempt by the processor to fit it into a preconceived mould.

The majority of implantees now implanted with a cochlear implant are able to understand speech to a significant degree without the aid of lip-reading. This is especially true when there has only been a brief period of time between the onset of deafness and the implantation in adult implantees. Even more importantly, studies suggest that in children there is only a restricted time window that allows best results with the cochlear implant, thus showing that implantation should be performed as early as possible. Despite the success of cochlear implants, studies have indicated there is variation in the level of success even when comparing the results of comparable subjects.

One posited explanation for the individual variability is the number of spiral ganglion cells still present in the cochlea of the implantee at the time of implantation. For example, following deafness, it is known that firstly, hair cells are lost and secondly, peripheral processes (dendrites) of the auditory nerve degenerate. Although not fully investigated, it is suggested that this leads to the loss of spiral ganglion cells (SGC) that represent the cell bodies of the auditory nerve located in the modiolus.

One proposal for improving the performance of cochlear implants is to actively prevent loss of spiral ganglion cells. Recently, chemical substances have been discovered that show a protective effect on spiral ganglion cells following onset of deafness in order to preserve the number of excitable units in the acoustic nerve. These so-called neurotrophic factors (NFs) include a wide variety of different substances, eg. GDNF, BDNF, NT-3, FGF, LIF and many others.

Delivery of drugs into the cochlea to achieve desired therapeutic results has been proposed in US Patent No 6,309,410. In that patent, there is described a cochlear implant electrode design that incorporates a dedicated drug delivery channel into the mechanics of the electrode carrier. The described system provides a channel extending through the length of the carrier which can be connected to a drug delivery pump or syringe to enable the drug to be delivered through the carrier. Such a design requires a significant increase in carrier size and complexity of design, to accommodate and manufacture this additional drug delivery channel. In the event that an additional electrode positioner is employed to force the carrier into position against the modiolar wall, such a system would also quickly fill up the available space in the cochlea resulting in a system which would potentially damage the sensitive structures of the cochlea and one that would be limited with regard to the depth of insertion due to the increased cross sectional area of the device.

An implantable system for infusing a fluid agent and providing electrical stimulation to a body using a source of fluid pressure and a source of electrical energy both located external to the body is known from US 5,697,951. The prior system includes a catheter which defines a fluid lumen for transporting a fluid agent through the catheter. The catheter comprises at least one electrode for administering electrical stimulation to a predetermined portion of the body, at least one catheter conductor for transmitting the stimulation pulses to the at least one electrode, and means for transporting the fluid agent from the fluid lumen to a predetermined portion of the body.

In conformity with WO 02/41666 A1 which constitutes prior art according to Article 54, paragraph 3 EPC, a cochlear implant electrode assembly comprises a pre-curved elongate flexible carrier which is adaptable to be inserted into the scala tympani of a human cochlea. A multiplicity of electrode contacts is exposed on a surface of the flexible carrier. A lumen extends through the carrier member for a portion of the length thereof. A stylet is positionable within the lumen and is adapted to bias the carrier member into a substantially straight configuration prior to insertion of the carrier member into the cochlea. The system is provided with an additional lumen that acts as a reservoir for a fluid constituting or including one or more pharmaceutical or bioactive substances. The lumen may be sealed following pre-filling, but also may be in fluid communication with an additional reservoir for the fluid. This additional reservoir can be placed under the skin of the implantee and be fillable by a needle and syringe assembly, and a pump, such as an osmotic pump, can be used to transfer fluid from the additional reservoir to the lumen. The lumen for the stylet can be the same as utilized in the substance delivery means.

The present applicant has developed a cochlear implant electrode array that is preferably capable of achieving a final implanted position that is close to the inner wall of the cochlea, without the need for any additional and space filling positioner elements as is the case with other commercially available products such as that referred to above. This implant array is pre-shaped into a curved configuration that closely resembles the natural shape of the cochlea, with the body of the carrier being formed with a lumen extending therethrough. The array is straightened and maintained in a straight configuration by a stylet element that is inserted into the lumen. Following implantation, the stylet is fully removed allowing the electrode array to relax and return to the shape matching the curvature of the cochlea thereby assuming a final position close to the inner wall of the cochlea and close to the desired stimulation sites. This design is described in US Patent Application 6,421,569.

The present application is directed to an improved system for delivering desired bioactive substances to the cochlea.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Summary of the Invention

The invention comprises a cochlear implant electrode assembly as defined in claim 1.

The cochlear implant electrode assembly comprising:
a pre-curved elongate flexible carrier member (11) adaptable to be inserted into the scala tympani of a human cochlea;
a multiplicity of electrode contacts (12) exposed on a surface of the flexible carrier member;
a chamber or lumen (14) extending through the carrier member for at least a portion of the length thereof; said chamber,or lumen being adapted to act as a drug delivery channel through which a bioactive fluid can move from a location external the carrier member to said chamber or lumen; and
means for interconnecting said chamber or lumen and said location external the carrier member;
characterized by a stiffening element (15) positionable within the chamber or lumen (14) and adapted to bias the carrier member into at least a substantially straight configuration prior to and during insertion of the carrier member into the cochlea;
wherein, following removal of the stiffening member from said chamber or lumen, said chamber or lumen is adapted to act as said drug delivery channel; and
by said interconnecting means comprising a connector device (50) having a first portion (52) that can interconnect with a first pipe (51) extending from said external location or an orifice in a reservoir or pump device (23), and a second portion (53) that can interconnect with a second pipe (59) extending from said chamber or lumen (14) or an orifice (17) in said chamber or lumen, wherein the first portion (52) has a larger outer diameter than the second portion (53).

The provision of a lumen to receive a straightening stylet provides a channel extending through the electrode array that can be adapted for delivery of pharmaceuticals to the cochlea following withdrawal of the stylet, without the need for an additional dedicated drug delivery channel. Such an auxiliary use of this lumen also simplifies the manufacture of the electrode array and allows only minor changes to the present manufacturing process to be made to achieve the improved result.

The outer diameter of the first portion can be about twice that of the outer diameter of the second portion. Still further, the outer diameter of the first portion can be between about 1 and 4 times greater, more preferably about 2.6 times greater, than the outer diameter of the second portion.

In a further embodiment, the outer diameter of the first portion can be substantially constant for at least a majority of its length. Still further, the outer diameter of the second portion can be substantially constant for at least a majority of its length. The outer diameter of the connector preferably steps outwardly from the outer diameter of the second portion to the outer diameter of the first portion and is provided by a step in the outer surface of the connector. The step in the outer surface of the connector can be at right angles to the longitudinal axis of the connector. In another embodiment, the step in the outer surface can comprise a fiusto-conical portion. Other geometries can also be envisaged.

In a preferred embodiment, the outer diameter of the first portion can be larger than the inner diameter of the first pipe, or the opening in the reservoir, or the outlet of the pump. Still further, the outer diameter of the second portion can be larger than the inner diameter of the second pipe, or the opening of the chamber or lumen of the carrier member. The respective larger outer diameters of the first and second portions as just described preferably serve to ensure that the frictional engagement made by the first and second portions of the connector when in use maintains the connector in place as a fluid transfer device in the device. The frictional engagement is preferably such that there are no or at least relatively minimal leaks of bioactive fluid when the fluid is being transferred through the connector.

In yet a further embodiment, a region of the first portion adjacent an outer end of the first portion can have a frusto-conical surface in which the diameter of the first portion tapers towards its outer end. A region of the second portion adjacent an outer end of the second portion can have a frusto-conical surface in which the outer diameter of the second portion tapers towards its outer end. Each outer end preferably has a relatively blunt aspect adapted to minimise the prospect of damage to the pipes, outlets or openings that are engaged to the respective portions of the connector.

The outer surface of the first and/or second portion can have one or more grooves formed therein adapted to enhance the frictional engagement between the connector and the pipes, openings or outlets connected thereto.

In a still further embodiment, the connector has a lumen extending therethrough between the respective outer ends of the first and second portions. In a preferred embodiment, the lumen has a constant inner diameter extending the length of the lumen. In a further embodiment, the lumen has an inner diameter about 50% that of the outer diameter of the second portion.

In a preferred embodiment, the lumen of the connector has an inner diameter of about 150 microns. The outer diameter of the first portion is preferably about 800 microns. The outer diameter of the second portion is preferably about 300 microns. The connector preferably has a length of about 8mm. The connector is preferably manufactured from a biocompatible material. In one embodiment, the connector can be manufactured from a metal or metal alloy, such as platinum or stainless steel. In another embodiment, the connector may be manufactured from a plastics or ceramic material.

In a further embodiment, the lumen of the connector can be adapted to receive a stiffening or straightening element that can pass through the connector and into the carrier member.

In one embodiment, the first pipe, or the reservoir opening, or the pump outlet, can be removably attachable to the first portion of the connector. In another embodiment, the second pipe, or the opening of the chamber or lumen of the carrier member, can be removably attachable to the second portion of the connector. In one embodiment, the second portion can be engaged with the second pipe, or the opening of the chamber or lumen of the carrier member, during the manufacturing and/or packaging process for the device and then delivered to the surgeon ready for use. In this arrangement, a stiffening or straightening element, such as a stylet, can also be prepackaged in the device and extend through the connector and into the carrier member.

In a still further embodiment, the first portion of the connector can be engaged with the first pipe, or the opening of the reservoir, or the outlet of the pump, during the surgical implantation procedure for the device. Such engagement preferably involves the surgeon sliding the first pipe or the opening or the outlet over the outer surface of the first portion to ensure frictional engagement therebetween. Where a stiffening or straightening element, such as a stylet, was packaged in the device, the carrier member would preferably be implanted in the cochlea, and the stylet removed, prior to engagement of the first pipe, or the opening, or the outlet, with the first portion of the connector.

While the outer surface of the respective first and second portions are described above as frictionally engaging with pipes, surfaces, or openings, it will be appreciated that the first and/or second pipes could be inserted into the lumen of the connector at the respective outer ends of the connector and so frictionally engaged with the surface of this lumen.

In one embodiment, the first pipe extends from a reservoir to the first side of said connector. The reservoir is preferably adapted to be implantable in the implantee receiving a cochlear implant device. In one embodiment, the reservoir can be implantable in the temporal bone of an implantee. In this embodiment, a pump is preferably used to draw fluid from the reservoir and transfer it through to the connector to the receiving location within the carrier member. Such a pump can also be implantable in the temporal bone of the implantee. In another embodiment, the pump can be implantable in soft tissue. In a further embodiment, the reservoir and/or pump can be explantable from the implantee if and when required. For example, the reservoir may need replacing or refilling to ensure bioactive fluid continues to be transferred to the receiving location in said carrier member. In another embodiment, an external body-worn pump having a trans-cutaneous connection could be utilised.

In a further embodiment, the first pipe extends from a pump to the first side of the connector. In one embodiment, the pump can comprise an osmotic pump that is implantable in soft tissue. In another embodiment, the pump can comprise an implantable and refillable electro-mechanical pump. In a further embodiment, the pump would be explantable from the implantee if and when required. For example, the pump may need replacing or refilling to ensure bioactive fluid continues to be transferred to the receiving location in said carrier member.

In a still further embodiment, the second pipe extends from a second side of the connector to at least the carrier member. In a preferred embodiment, the second pipe extends from the second side of the connector to an opening of a lumen within the carrier member, said lumen acting as a fluid receiving location within the carrier member.

In one embodiment the lumen of the carrier member can extend through the carrier member for at least a portion of the length of the carrier member. In a further embodiment, the lumen extends through the carrier member for a majority, and preferably a substantial portion, of the length of the carrier member. In a further embodiment, the second pipe can be integrally formed with the body of the carrier member.

It will be appreciated that the features of the lumen of the carrier member defined herein can be present whether a second pipe interconnects the second portion of the connector with the lumen opening or whether the second portion is directly connected to the opening of the lumen of the carrier member.

In a further embodiment, the carrier member has a leading end that is insertable into a cochlea of an implantee and a trailing end distal the leading end. In this embodiment, the lumen preferably extends from an opening at or adjacent the trailing end to a position adjacent the leading end.

The elongate carrier member preferably has a plurality of electrodes mounted thereon. In one embodiment, the electrodes are mounted in a longitudinal array. Each of the electrodes have at least one wire, and preferably at least two, extending from each electrode back towards the trailing end of the carrier member.

The elongate carrier member is preferably formed from a resiliently flexible material. In one embodiment, the carrier member can be preformed from a plastics material with memory.

In a preferred embodiment, the orientation of the carrier member as it is firstly inserted through a cochleostomy into the cochlea is preferably substantially straight. More preferably, the implantable orientation is straight. Following completion of implantation, the carrier member preferably adopts a spirally curved configuration that matches the spiral nature of the scala tympani of the human cochlea. The carrier member is preferably pre-formed with this spiral configuration and is then straightened either during manufacture and packaging of the device or prior to implantation.

In a preferred embodiment, the elongate carrier member is formed from a suitable biocompatible material. In one embodiment, the biocompatible material can be a silicone, such as a flexible silicone elastomer-Silastic. Silastic MDX 4-4210 is an example of one suitable silicone for use in the formation of the elongate member. In another embodiment, the elongate carrier member can be formed from a polyurethane or similar material.

The cochlear implant electrode assembly device preferably further comprises at least one stiffening or straightening element that is adapted to bias the carrier member into at least a substantially straight configuration prior to and during initial insertion of the carrier member through the cochleostomy. The stiffening or straightening element is preferably positioned during manufacture of the device and serves to straighten the carrier member that would otherwise preferentially adopt a spirally curved configuration. As discussed above, the stiffening element can extend through the lumen of the connector.

The stiffening or straightening element is preferably formed of a material that is relatively stiffer than the material of the carrier member.

The stiffening or straightening element is preferably formed from a non-bioresorbable material. In this embodiment, the stiffening or straightening element can comprise a metallic or plastic stylet. The stylet can extend through a lumen extending within at least a portion of the length, more preferably a majority of the length, of the elongate carrier member. In one embodiment, the stylet can extend into the lumen adapted to act as the fluid receiving location of the carrier member as defined above.

In one embodiment, the stylet can be formed from a biocompatible material, such as a metal or metallic alloy. In a preferred embodiment, the metal stylet is formed from platinum.

The lumen can be cylindrical or have another cross-sectional shape. In one embodiment, the lumen preferably has a diameter of about 180 microns. In the case where the metal stylet is used, the stylet can extend out of the opening at or adjacent the trailing end so allowing the stylet to be manipulated and removed from the lumen during the insertion procedure of the carrier member.

The lumen when acting as said fluid receiving location in the elongate carrier member can act as a reservoir for a bioactive fluid within the elongate member. In one embodiment, the carrier member can be formed of a material such that the bio-active fluid when in the reservoir can leach from the lumen into the surrounding material of the elongate carrier member and eventually migrate to an outer surface of the member that is preferably close to the desired site of action for the bio-active fluid. In another embodiment, the elongate member can have one or more fluid egress paths whereby the fluid can move out of the lumen and the elongate carrier member to a position that is preferably close to the desired site of action for the bio-active fluid.

Each fluid egress paths can have a valve that allows fluid to exit the lumen but prevents, or at least substantially prevents, fluid flow from external the elongate carrier member back into the lumpen within the elongate carrier member.

In a further embodiment, the bioactive fluid comprises one or more neurotrophic factors. In a preferred embodiment, the neurotrophic factor can be selected from the group comprising GDNF, BDNF, NT-3, FGF, and LIF.

Once implanted, the electrodes of the carrier member of the above aspects preferably receive stimulation signals from a stimulator. The stimulator is preferably electrically connected to the elongate carrier member by way of an electrical lead. The lead can include the one or more wires extending from each electrode of the array mounted on the elongate member.

In one embodiment, the lead can extend from the elongate member to the stimulator or at least the housing thereof. In one embodiment, the lead is continuous with no electrical connectors, at least external the housing of the stimulator, required to connect the wires extending from the electrodes to the stimulator. One advantage of this arrangement is that there is no requirement for the surgeon implanting the device to make the necessary electrical connection between the wires extending from the electrodes and the stimulator.

The stimulator is preferably positioned within a housing that is implantable within the implantee. The housing for the stimulator is preferably implantable within a recess in the bone behind the ear posterior to the mastoid.

When implanted, the housing preferably contains, in addition to the stimulator, a receiver unit. The receiver unit is preferably adapted to receive signals from a controller. The controller is, in use, preferably mounted external to the body of the implantee such that the signals are transmitted transcutaneously through the skin of the implantee.

Signals can preferably travel from the controller to the receiver unit and vice versa. The receiver unit can include a receiver coil adapted to receive radio frequency (RF) signals from a corresponding transmitter coil worn externally of the body. The radio frequency signals can comprise frequency modulated (FM) signals. While described as a receiver coil, the receiver coil can preferably transmit signals to the transmitter coil which receives the signals.

The transmitter coil is preferably held in position adjacent the implanted location of the receiver coil by way of respective attractive magnets mounted centrally in, or at some other position relative to, the coils.

The external controller can comprise a speech processor adapted to receive signals output by a microphone. During use, the microphone is preferably worn on the pinna of the implantee, however, other suitable locations can be envisaged, such as a lapel of the implantee's clothing. The speech processor encodes the sound detected by the microphone into a sequence of electrical stimuli following given algorithms, such as algorithms already developed for cochlear implant systems. The encoded sequence is transferred to the implanted stimulator/receiver unit using the transmitter and receiver coils. The implanted stimulator/receiver unit demodulates the FM signals and allocates the electrical pulses to the appropriate attached electrode by an algorithm which is consistent with the chosen speech coding strategy.

The external controller further comprises a power supply. The power supply can comprise one or more rechargeable batteries. The transmitter and receiver coils are used to provide power via transcutaneous induction to the implanted stimulator/receiver unit and the electrode array.

While the implant system can rely on external componentry, in another embodiment, the controller, including the microphone, speech processor and power supply can also be implantable. In this embodiment, the controller can be contained within a hermetically sealed housing or the housing used for the stimulator.

### Brief Description of the Drawings

By way of example only, a preferred embodiment of the invention is now described with reference to the accompanying drawings, in which:
Fig. 1 is a pictorial representation of a prior art cochlear implant system;
Fig. 2 is a cross-sectional view of a connector according to the present invention;
Fig. 3 is a view of the connector of Fig. 2 with a stylet extending through the connector;
Fig. 4 is a simplified cross-sectional view of one embodiment of a carrier member for a cochlear implant electrode assembly device; and
Fig. 5 is a view of the carrier member following insertion in the cochlea, with the connector in use connecting the output of a pump to the lumen of the carrier member.

### Preferred Mode of Carrying out the Invention

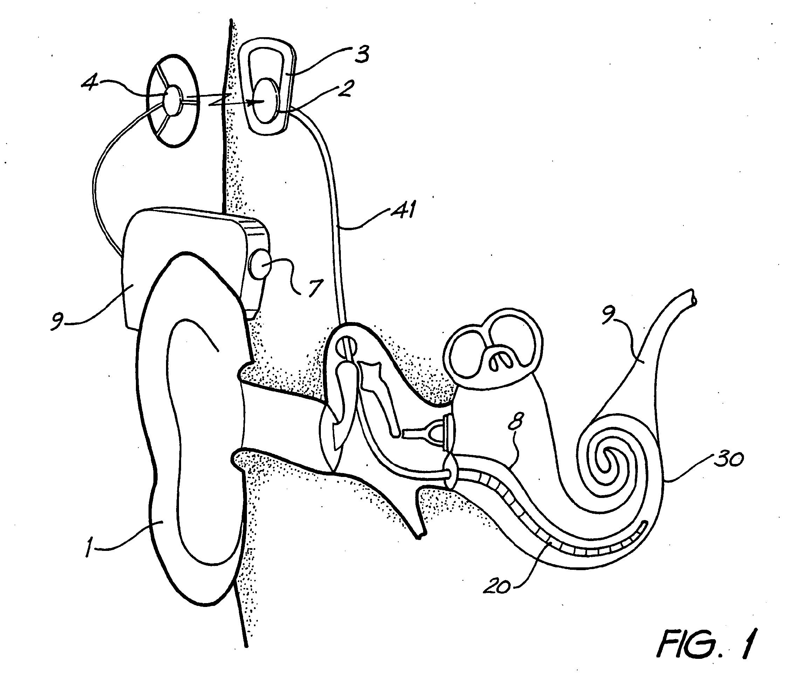

Before describing the features of the present invention, it is appropriate to briefly describe the construction of one type of known cochlear implant system with reference to Fig. 1.

Known cochlear implants typically consist of two main components, an external component including a speech processor 9, and an internal component including an implanted receiver and stimulator unit 2. The external component includes a microphone 7. The speech processor 9 is, in this illustration, constructed and arranged so that it can fit behind the outer ear 1. Alternative versions may be worn on the body. Attached to the speech processor 9 is a transmitter coil 4 which transmits electrical signals to the implanted unit 2 via a radio frequency (RF) link.

The implanted component includes a receiver coil 3 for receiving power and data from the transmitter coil 4. A cable 41 extends from the implanted receiver and stimulator unit 2 to the cochlea 30 and terminates in an electrode array 20. The signals thus received are applied by the array 20 to the basilar membrane 8 and the nerve cells within the cochlea 30 thereby stimulating the auditory nerve 9. The operation of such a device is described, for example, in US patent No. 4532930.

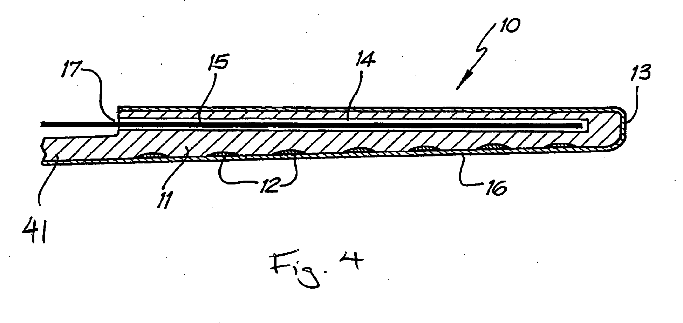

One embodiment of a cochlear implant electrode assembly according to the present invention is depicted generally as 10 in Fig. 4. This assembly can, if desired, be used in conjunction with a connector as is also described herein.

The depicted electrode assembly 10 has an electrical lead 41 extending back to a stimulator/receiver housing, such as the stimulator unit 2 depicted in Fig. 1. In considering this invention, it is to be understood that each electrode 12 may have one or more wires (not depicted) electrically connected thereto and extending from each respective electrode 12 back through the lead 41 to the stimulator/receiver unit.

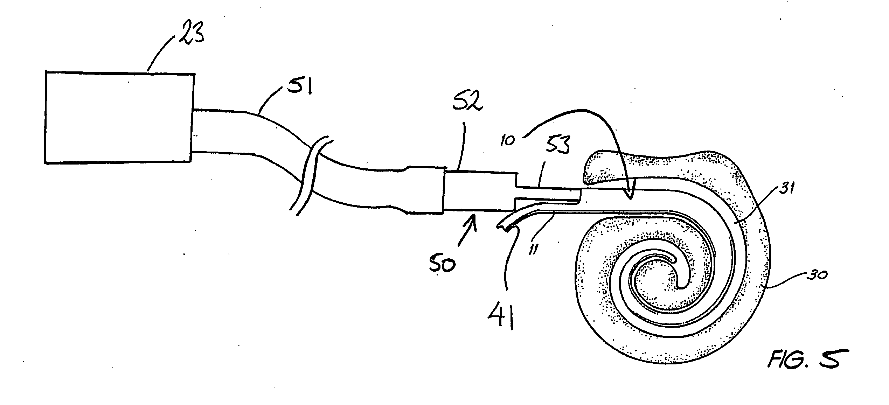

The assembly 10 comprises an elongate electrode carrier member 11 having a plurality of electrodes 12 mounted thereon. For the purposes of clarity, the electrodes 12 depicted in Fig. 4 are not necessarily shown to scale. A larger number of electrodes than that depicted in Fig. 4 can also be envisaged. The electrodes 12 are not depicted in Fig. 5 for reasons of clarity. The depicted carrier member 11 can have many of the features of a carrier member of a Nucleus 24 Contour electrode array (Cochlear Ltd, Sydney, Australia). Such an array is self-curling and designed for placement close to the modiolus within the scala tympani. This array is described in more detail in US Patent No. 6,421,569.

The depicted elongate member 11 is preformed from a resiliently flexible silicone (Silastic) with memory and is preformed to a curved configuration suitable for insertion in the scala tympani 31 of a human cochlea 30. While an assembly that normally adopts a curved configuration when in a relaxed condition is depicted in the drawings, it will be appreciated that the present invention also could be utilised with respect to assemblies that are normally straight when in a relaxed condition.

The elongate member 11 has a first end 13 that is firstly inserted into the cochlea 30 upon insertion of the assembly 10.

The depicted carrier member has an outer layer 16 of a lubricious material. The lubricious material can be selected from the group comprising polyacrylic acid (PAA), polyvinyl alcohol (PVA), polylactic acid (PLA), and polyglycolic acid (PGA). The lubricious material is preferably resorbed relatively quickly once the carrier member 11 has been implanted in the cochlea 30. It will be appreciated that the carrier member 11 could be used without the layer 16 depicted in Fig. 4.

As depicted in Fig. 4, there is disposed within a lumen 14, prior to insertion of the assembly 10 into the cochlea 30, a substantially straight platinum stylet 15. In the depicted embodiment, the stylet 15 has a stiffness that is sufficient to retain the silicone elongate member 11 in a straight configuration. The stylet can have a diameter of about 180 microns and reach close to the leading end 13 of the carrier member 11.

In the depicted embodiment, the elongate member 11 is adapted to also act as a system for delivery of one or more pharmaceutical or bioactive substances to the cochlea 30. In this regard, the elongate carrier member is preferably adapted to deliver a bioactive fluid comprising one or more neurotrophic factors, such as any one or more of GDNF, BDNF, NT-3, FGF, LIF or other such factors.

In the depicted embodiment, a lumen 14 whilst acting to receive a stylet can also be used as a chamber or reservoir for a fluid constituting or including the one or more pharmaceutical or bioactive substances. As is depicted in Fig. 5, the lumen 14 is in fluid communication with a pump 23. The pump 23 can be placed under the skin of the implantee and be fillable or replaceable as required. The depicted pump is an osmotic pump. While the use of such a pump for this application in humans will require regulatory approval, an example of a type of pump that might be utilised, with suitable modification, is the Alzet mini-osmotic pump (Model 2002, Alza Corp, Palo Alto, California, USA).

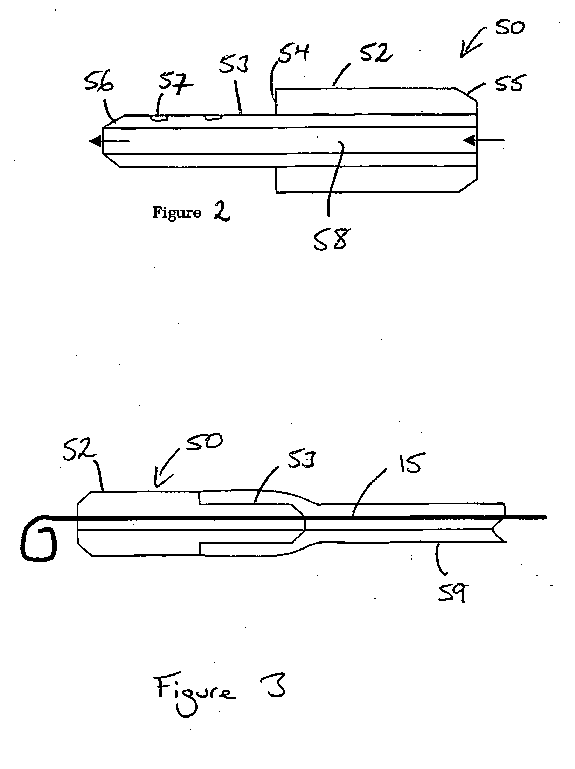

One example of a connector for providing fluid communication between the output of the pump 23 and the opening 17 of the lumen 14 of the carrier member 11 is depicted generally as 50 in Figs. 2, 3 and 5.

As is depicted in Fig. 5, the connector 50 can provide a fluid transfer device between a first pipe 51 extending from the pump 23 and the opening 17 of the lumen 14 of the carrier member 11. While not depicted, the connector 50 can be readily envisaged providing a fluid transfer device between the first pipe 51 extending from said external location and a second pipe that extends from the connector 50 to the opening 17. Still further, the first pipe 51 could be omitted with the connector 50 interconnecting an opening in a reservoir, or an outlet of the pump 23, with a second pipe, or the opening 17 of the lumen 14 in the carrier member 11.

The depicted connector 50 has a first portion 52 connectable to the first pipe 51 as depicted in Fig. 5, or as discussed, directly with an opening in the reservoir, or the outlet of the pump 23. The connector 50 also has a second portion 53 connectable to the opening 17 as depicted in Fig. 5, or as discussed, with a second pipe extending from the connector 50 to the opening 17.

As depicted, the first portion 52 and second portion 53 of the connector 50 are cylindrical for the majority of their respective lengths and have different outer diameters, with the first portion 52 having a larger outer diameter than the second portion 53. In the depicted embodiment, the outer diameter of the first portion 52 is about 800 microns and the outer diameter of the second portion 53 is about 300 microns. A step 54 is provided in the outer surface of the connector 50 between the respective portions.

The outer diameter of the first portion 52 is larger than the inner diameter of the first pipe 51 as is depicted in Fig. 5. The pipe 51 can be formed of a silicone rubber and have a diameter of about 650 microns and a length of about 11.7 cm. The slidable engagement between the first portion 52 and the first pipe 51 preferably extends for a distance of about 5mm. The outer diameter of the second portion 53 is also larger than the diameter of the opening 17 of the lumen 14 of the carrier member 11. The respective larger outer diameters of the first and second portions as just described serve to ensure that the frictional engagement made by the first and second portions of the connector 50 when in use maintains the connector 50 in place as a fluid transfer device in the device. The frictional engagement is preferably such that there are no or relatively minimal leaks of bioactive fluid when the fluid is being transferred through the connector 50 from the pump 23 to the lumen 14.

As depicted in Fig. 2, the outer end of the first portion 52 has a frusto-conical surface 55 in which the diameter of the first portion 52 tapers towards its outer end. The outer end of the second portion 53 also has a frusto-conical surface 56 in which the outer diameter of the second portion 53 tapers towards its outer end. Each outer end also has a relatively blunt aspect adapted to minimise the prospect of damage to the pipes, outlets or openings that are engaged to the respective portions of the connector 50.

As is at least partially depicted in Fig. 2, the outer surface of the first and/or second portions can have one or more grooves 57 formed therein and adapted to enhance the frictional engagement between the connector 50 and the pipes, openings or outlets connected thereto.

The connector 50 also has a lumen 58 extending therethrough between the respective outer ends of the first and second portions. The lumen 58 has a constant inner diameter extending the length of the lumen. The inner diameter of the lumen 58 is about 50% that of the outer diameter of the second portion 53 and is about 150 microns. The depicted connector has a length of about 8mm and is manufactured from a biocompatible material such as platinum or stainless steel.

As depicted in Fig. 3, the lumen 58 of the connector 50 can receive a stylet 15 that passes through the connector 50 and into the lumen 14 of the carrier member 11. In this depicted embodiment, the second portion 53 is engaged with a second pipe 59 that is engaged with the second portion 53 and extends into and sealingly engages the opening 17 of the lumen 14.

It is envisaged that there are a number of techniques that could be employed to both implant the connector and ensure flow of bioactive fluid from the pump 23 to the lumen 14 of the carrier member 11. In one technique, the stylet 15 can firstly extend through the connector 50 (as depicted in Fig. 3) and into the lumen 14. The carrier member 11 can then be inserted into and placed within the cochlea 30 in a position as is depicted in Fig. 5. During or after placement, the stylet 50 can be withdrawn from the lumen 14 and the connector 50. Once the stylet 50 is removed, the surgeon can slide the end of the first pipe 51 over the first portion 52. In this case, it would be envisaged that the pump 23 would be implanted prior to connection of first pipe 51 to the first portion 52.

In another technique, the carrier member 11 could be placed in the cochlea 30. In one technique, this may involve use of a stylet 15 but not with the stylet extending through the connector 50 as is depicted in Fig. 3. After placement of the carrier member 11, the second portion 53 of the connector can be slidably inserted by the surgeon into the opening 17 of the lumen 14. A fine pair of forceps could be used to perform this action. In this technique, the first pipe 51 could be already connected to the first portion 52 of the connector 50 prior to placement of the carrier member 11 in the cochlea 30. Alternatively, such a connection could be made after the second portion 53 has been engaged with the lumen 14.

As depicted in Fig. 4, the lumen 14 of the carrier member 11 can extend through the carrier member 11 for a substantial portion of its length.

The carrier member 11 is formed from a material such that the bioactive fluid can leach from the elongate member 11 following its implantation. One or more relatively small openings (not depicted) can be provided in the carrier member 11 to allow the fluid to relatively readily exit the lumen 14 following implantation of the carrier member 11 and operation of pump 23.

While the elongate member 11 is manufactured with a preformed curved configuration, the depicted assembly 10 is typically delivered to a surgeon in a sterile package with the stylet 15 in place (as depicted in Fig. 4).

As the elongate member 11 is inserted into the scala tympani 31 of the cochlea 30, the surgeon can commence to withdraw the stylet 15 from the lumen 14 through opening 17. On withdrawal of the stylet 15, the elongate member 11 is free to adopt the spiral configuration depicted in Fig. 5 with the electrodes 12 facing the modiolus within the cochlea 30 so that they are positioned as close as possible to the spiral ganglia thereof.

### Experiments

### Methods

Experiments have been performed on the connector 50 to test for mechanical stability. In these tests, tractive forces were applied to the connection made by the second portion 53 and the lumen 14 and the first portion 52 and the first pipe 51.

In the experiments a dial tension gauge (DTG-100P, Mitutoyo Corp., Kanagawa, Japan) was used to measure tractive forces needed to disconnect the connector 50. The experiment was conducted using five unused Contour electrode arrays (Cochlear Ltd, Sydney, Australia).

In addition, experiments were also performed using an Alzet mini-osmotic pump (Model 2002) in order to investigate the feasibility of the lumen 14 as a reservoir for bioactive fluid drug delivery. The flow rate provided by this pump was 0.5 µl per hour (maximal pump duration 14 days). The pumps were prepared as recommended by the manufacturer's guidelines and filled with methylene blue dye (Methylenblau vitis 1%, Neopharma, Aschau, Germany). The flow moderators of the pumps were connected to the tubing and the system placed for three days into a physiological saline solution constantly heated to 37°C.

To appropriately test the connector 50, the experiments also involved use of a high flow rate for short term experiments or a low flow rate (100 µl/h), both created by an infusion pump (Perfusor VII, B. Braun, Melsungen, Germany).

For acute short-term experiments, the infusion pump was filled with a methylene blue dye solution. Delivery rates were gradually increased from 0.5 to 20 ml/h up to 20 min. The seal between the connector 50 and the lumen 14 was monitored by visual inspection and in addition by the placement of absorbent paper beneath the connector 50, thereby making fluid leakage immediately detectable to the observer.

Further experiments were also performed using the infusion pump at a pump rate of 100 µl/h for 15 days. During these experiments, the whole system from the pump to the electrode carrier member 11 array was lying on absorbent paper and the very tip of the array hung above a beaker.

Two human temporal bones were also used to test for feasibility of the insertion and connecting procedures for a system as depicted in Fig. 5. The bones were dissected following common techniques and a cochleostomy was performed through the facial recess. The carrier member 11 was inserted into the scala tympani 31 using standard insertion techniques. Following insertion, the stylet 15 was removed while holding the carrier member 11 in place with fine forceps. The connector 50 was connected to an infusion pump (pump rate 1 ml/h) as described above. Then, the second portion 53 of the connector 50 was inserted into the lumen 14 of the carrier member 11 using fine forceps. The pump and carrier member 11 were then filled with contrast dye (imeron 300, Byk-Gulden, Konstanz, Germany) and continuous X-ray radiography (Neurostar, Siemens AG, Forchheim, Germany) was used to visualise fluid entry into the cochlea 30. Single screen shots were taken to document results.

### Results

The experiments determined that the mean tractive force needed to pull apart either the connector 50 from the lumen 14 of the carrier member 11 or the tubing or pipe 51 from the connector 50 was on average 56N (±7N).

When tested for patency of the connection area, experiments were conducted with continuous fluid flow of the dye through the system. The only fluid exit was observed from the carrier member 11 as was expected. There was no fluid leakage from the connections made with the connector 50. This result was the same when using an Alzet mini-osmotic pump (0.5 µl/h) or the mechanic infusion pump with pump rates from 0.5 to 20 ml/h up to 20 min. Even when the system was tested for a period of 15 days with an infusion pump (flow rate 0.1 ml/h), no fluid leakage was observed from the connections made with the connector 50.

The temporal bone studies revealed that the modified carrier members 11 could be inserted into the cochlea 30 as easily as standard carrier members, such as the Nucleus Contour electrode array (Cochlear Ltd, Sydney, Australia). The stylet 15 proved to be easily removable through the connector 50. Still further, all necessary connections to the connector 50 could be made under surgical conditions. The experiments also showed that the contrast dye delivered to the cochlea 30 could be detected using X-ray radiography, and that fluid transport into the scala tympani 31 was possible using the system depicted in Fig. 5.

The experiments conducted using the connector 50 reveal that the lumen 14 of the carrier member 11 can be used as a means of delivering bioactive fluids to the cochlea 30 of a human subject. The stylet lumen even though only typically 180 µm in diameter, can have fluid flow therethrough created by a mini-osmotic pump (0.5 □l/h) or a commonly used infusion pump.

The provision of a system, incorporating the connector 50, for delivering a bioactive substance that promotes healing and/or more efficient neural stimulation while preventing the formation of substantial scar tissue in the cochlea, enhances the likelihood of successful long-term placement of the assembly 10 in the cochlea and subsequent successful use of the cochlear implant by the implantee.

It will be appreciated by persons skilled in the art that numerous variations and/or modifications may be made to the invention as shown in the specific embodiments without departing from the scope of the invention as defined in claim 1. The present embodiments are, therefore, to be considered in all respects as illustrative and not restrictive.

## Claims

1. A cochlear implant electrode assembly comprising:
a pre-curved elongate flexible carrier member (11) adaptable to be inserted into the scala tympani of a human cochlea;
a multiplicity of electrode contacts (12) exposed on a surface of the flexible carrier member;
a chamber or lumen (14) extending through the carrier member for at least a portion of the length thereof; said chamber or lumen being adapted to act as a drug delivery channel through which a bioactive fluid can move from a location external the carrier member to said chamber or lumen; and
means for interconnecting said chamber or lumen and said location external the carrier member;
**characterized by** a stiffening element (15) positionable within the chamber or lumen (14) and adapted to bias the carrier member into at least a substantially straight configuration prior to and during insertion of the carrier member into the cochlea;
wherein, following removal of the stiffening member from said chamber or lumen, said chamber or lumen is adapted to act as said drug delivery channel; and
by said interconnecting means comprising a connector device (50) having a first portion (52) that can interconnect with a first pipe (51) extending from said external location or an orifice in a reservoir or pump device (23), and a second portion (53) that can interconnect with a second pipe (59) extending from said chamber or lumen (14) or an orifice (17) in said chamber or lumen, wherein the first portion (52) has a larger outer diameter than the second portion (53).

2. A cochlear implant electrode assembly as claimed in claim 1 wherein the outer diameter of the first portion (52) is about twice that of the outer diameter of the second portion (53).

3. A cochlear implant electrode assembly as claimed in claim 1 wherein the outer diameter of the first portion (52) is between about 1 and 4 times greater than the outer diameter of the second portion (53).

4. A cochlear implant electrode assembly as claimed in claim 1 wherein the outer diameter of the first portion (52) is about 2.6 times greater than the outer diameter of the second portion (53).

5. A cochlear implant electrode assembly as claimed in any one of the preceding claims wherein the outer diameter of the first portion (52) is substantially constant for at least a majority of its length.

6. A cochlear implant electrode assembly as claimed in claim 5 wherein the outer diameter of the second portion (53) is substantially constant for at least a majority of its length.

7. A cochlear implant electrode assembly as claimed in claim 6 wherein the outer diameter of the connector device (50) steps outwardly from the outer diameter of the second portion (53) to the outer diameter of the first portion (52) and is provided by a step (54) in the outer surface of the connector device.

8. A cochlear implant electrode assembly as claimed in claim 7 wherein the step (54) in the outer surface of the connector device (50) is at right angles to the longitudinal axis of the connector device.

9. A cochlear implant electrode assembly as claimed in any one of the preceding claims wherein the connector device (50) has a lumen (58) extending therethrough between the respective outer ends of the first and second portions (52, 53).

10. A cochlear implant electrode assembly as claimed in any one of the preceding claims wherein the reservoir is implantable in the implantee receiving a cochlear implant electrode assembly.

11. A cochlear implant electrode assembly as claimed in claim 10 further comprising a pump (23) that is adapted to transfer the bioactive fluid from the reservoir to said chamber or lumen (14) within the carrier member (11).

12. A cochlear implant electrode assembly as claimed in claim 11 wherein the pump (23) is implantable in the implantee.

13. A cochlear implants electrode assembly as claimed in any one of the preceding claims wherein the bioactive fluid comprises one or more neurotrophic factors selected from the group comprising GDNF, BDNF, NT-3, FGF, and LIF.

## Patentansprüche

1. Elektrodenanordnung für ein Cochlear-Implantat versehen mit:
einem vorgekrümmten, länglichen, flexiblen Trägerbauteil (11), welches anpassbar ist, um in die Paukentreppe einer menschlichen Cochlea eingeführt zu werden;
einer Mehrzahl von Elektrodenkontakten (12), die an einer Oberfläche des flexiblen Trägerbauteils freiliegen;
einer Kammer oder einem Lumen (14), welches sich durch das Trägerbauteil über mindestens einen Teil von dessen Länge erstreckt; wobei die Kammer oder das Lumen ausgelegt ist, als ein Medikamentenzufuhrkanal zu dienen, durch welchen sich ein bioaktiyes Fluid von einer bezüglich dem Trägerbauteil externen Stelle zu der Kammer oder dem Lumen bewegen kann; und
einer Anordnung zum Verbinden der Kammer oder des Lumens mit der bezüglich dem Trägerbauteil externen Stelle;
**gekennzeichnet durch** ein Versteifungselement (15), welches innerhalb der Kammer oder dem Lumen (14) angeordnet werden kann und welches ausgelegt ist, das Trägerbauteil in eine mindestens im Wesentlichen gerade Konfiguration vorzuspannen, bevor und während das Trägerbauteil in die Cochlea eingeführt wird; wobei im Anschluss an das Entfernen des Versteifungsbauteils von der Kammer oder dem Lumen die Kammer oder das Lumen ausgelegt ist, als der Medikamentenzufuhrkanal zu dienen; und
**dadurch**, dass die Verbindungsanordnung eine Verbindervorrichtung (50) aufweist mit einem ersten Bereich (52), der für eine Verbindung zu einer ersten Leitung (51) sorgen kann, die sich von der externen Stelle oder einer Öffnung in einem Reservoir oder einer Pumpvorrichtung (23) erstreckt, sowie einem zweiten Bereich (53), der für eine Verbindung zu einer zweiten Leitung (59) sorgen kann, die sich von der Kammer oder dem Lumen (14) oder einer Öffnung (17) in der Kammer oder dem Lumen erstreckt, wobei der erste Bereich (52) einen größeren Außendurchmesser als der zweite Bereich (53) hat.

2. Elektrodenanordnung für ein Cochlear-Implantat gemäß Anspruch 1, bei welcher der Außendurchmesser des ersten Bereichs (52) etwa doppelt so groß wie der Außendurchmesser des zweiten Bereichs (53) ist.

3. Elektrodenanordnung für ein Cochlear-Implantat gemäß Anspruch 1, bei welcher der Außendurchmesser des ersten Bereichs (52) zwischen etwa 1- und 4-mal so groß ist wie der Außendurchmesser des zweiten Bereichs (53).

4. Elektrodenanordnung für ein Cochlear-Implantat gemäß Anspruch 1, bei welcher der Außendurchmesser des ersten Bereichs (52) etwa 2,6-mal so groß ist wie der Außendurchmesser des zweiten Bereichs (53).

5. Elektrodenanordnung für ein Cochlear-Implantat gemäß einem der vorhergehenden Ansprüche, bei welcher der Außendurchmesser des ersten Bereichs (52) über mindestens einen Großteil seiner Länge im Wesentlichen konstant ist.

6. Elektrodenanordnung für ein Cochlear-Implantat gemäß Anspruch 5, bei welcher der Außendurchmesser des zweiten Bereichs (53) über mindestens einen Großteil seiner Länge im Wesentlichen konstant ist.

7. Elektrodenanordnung für ein Cochlear-Implantat gemäß Anspruch 6, bei welcher der Außendurchmesser der Verbindervorrichtung (50) von dem Außendurchmesser des zweiten Bereichs (53) zu dem Außendurchmesser des ersten Bereichs (52) nach außen abgestuft ist und durch eine Stufe (54) in der Außenfläche der Verbindervorrichtung bereitgestellt wird.

8. Elektrodenanordnung für ein Cochlear-Implantat gemäß Anspruch 7, bei welcher die Stufe (54) in der Außenfläche der Verbindervorrichtung (50) rechtwinklig bezüglich der Längsachse der Verbindervorrichtung ist.

9. Elektrodenanordnung für ein Cochlear-Implantat gemäß einem der vorhergehenden Ansprüche, bei welcher die Verbindervorrichtung (50) ein Lumen (58) aufweist, welches zwischen den betreffenden äußeren Enden des ersten und des zweiten Bereichs (52, 53) durch diese verläuft.

10. Elektrodenanordnung für ein Cochlear-Implantat gemäß einem der vorhergehenden Ansprüche, bei welcher das Reservoir in den Implantatträger, der eine Elektrodenanordnung für ein Cochlear-Implantat erhält, implantierbar ist.

11. Elektrodenanordnung für ein Cochlear-Implantat gemäß Anspruch 10, ferner versehen mit einer Pumpe (23), die ausgelegt ist, das bioaktive Fluid von dem Reservoir zu der Kammer oder dem Lumen (14) innerhalb des Trägerbauteils (11) zu übertragen.

12. Elektrodenanordnung für ein Cochlear-Implantat gemäß Anspruch 11, bei welcher die Pumpe (23) in den Implantatträger implantierbar ist.

13. Elektrodenanordnung für ein Cochlear-Implantat gemäß einem der vorhergehenden Ansprüche, bei welcher das bioaktive Fluid einen oder mehrere neurotrope Faktoren aufweist, die aus der Gruppe bestehend aus GDNF, BDNF, NT-3, FGF und LIF ausgewählt sind.

## Revendications

1. Ensemble d'électrode d'implant cochléaire, comprenant :
un élément de support souple allongé pré-incurvé (11) pouvant être adapté pour être inséré dans la rampe tympanique d'une cochlée humaine ;
une multiplicité de contacts d'électrode (12) exposés sur une surface de l'élément de support souple ;
une chambre ou conduit (14) s'étendant à travers l'élément de support sur au moins une partie de la longueur de celui-ci ; ladite chambre ou ledit conduit étant adapté de façon à jouer le rôle de canal de délivrance de médicament à travers lequel un fluide bio-actif peut se déplacer d'un emplacement extérieur à l'élément de support à ladite chambre ou audit conduit ; et
des moyens pour interconnecter ladite chambre ou ledit conduit et ledit emplacement extérieur à l'élément de support ;
**caractérisé par** un élément raidisseur (15) pouvant être positionné à l'intérieur de la chambre ou du conduit (14) et adapté de façon à solliciter l'élément de support dans au moins une configuration sensiblement droite avant et pendant l'insertion de l'élément de support dans la cochlée ;
dans lequel, après le retrait de l'élément raidisseur à partir de ladite chambre ou dudit conduit, ladite chambre ou ledit conduit est adapté de façon à jouer le rôle dudit canal de délivrance de médicament ; et
par le fait que lesdits moyens d'interconnexion comprennent un dispositif de connecteur (50) comportant une première partie (52) qui peut s'interconnecter avec un premier tuyau (51) s'étendant à partir dudit emplacement extérieur ou d'un orifice dans un dispositif de réservoir ou de pompe (23), et une deuxième partie (53) qui peut s'interconnecter avec un deuxième tuyau (59) s'étendant à partir de ladite chambre ou dudit conduit (14) ou d'un orifice (17) dans ladite chambre ou ledit conduit, la première partie (52) ayant un diamètre extérieur supérieur à celui de la deuxième partie (53).

2. Ensemble d'électrode d'implant cochléaire selon la revendication 1, dans lequel le diamètre extérieur de la première partie (52) est environ le double du diamètre extérieur de la deuxième partie (53).

3. Ensemble d'électrode d'implant cochléaire selon la revendication 1, dans lequel le diamètre extérieur de la première partie (52) est environ entre 1 et 4 fois plus grand que le diamètre extérieur de la deuxième partie (53).

4. Ensemble d'électrode d'implant cochléaire selon la revendication 1, dans lequel le diamètre extérieur de la première partie (52) est environ 2,6 fois plus grand que le diamètre extérieur de la deuxième partie (53).

5. Ensemble d'électrode d'implant cochléaire selon l'une quelconque des revendications précédentes, dans lequel le diamètre extérieur de la première partie (52) est sensiblement constant sur au moins une majorité de sa longueur.

6. Ensemble d'électrode d'implant cochléaire selon la revendication 5, dans lequel le diamètre extérieur de la deuxième partie (53) est sensiblement constant sur au moins une majorité de sa longueur.

7. Ensemble d'électrode d'implant cochléaire selon la revendication 6, dans lequel le diamètre extérieur du dispositif de connecteur (50) est étagé vers l'extérieur du diamètre extérieur de la deuxième partie (53) au diamètre extérieur de la première partie (52) et est constitué par un épaulement (54) dans la surface extérieure du dispositif de connecteur.

8. Ensemble d'électrode d'implant cochléaire selon la revendication 7, dans lequel l'épaulement (54) dans la surface extérieure du dispositif de connecteur (50) est à angle droit avec l'axe longitudinal du dispositif de connecteur.

9. Ensemble d'électrode d'implant cochléaire selon l'une quelconque des revendications précédentes, dans lequel le dispositif de connecteur (50) comporte un conduit (58) s'étendant à travers celui-ci entre les extrémités extérieures respectives des première et deuxième parties (52, 53).

10. Ensemble d'électrode d'implant cochléaire selon l'une quelconque des revendications précédentes, dans lequel le réservoir peut être implanté dans le patient recevant un ensemble d'électrode d'implant cochléaire.

11. Ensemble d'électrode d'implant cochléaire selon la revendication 10, comprenant de plus une pompe (23) qui est adaptée pour transférer le fluide bio-actif du réservoir à ladite chambre ou audit conduit (14) à l'intérieur de l'élément de support (11).

12. Ensemble d'électrode d'implant cochléaire selon la revendication 11, dans lequel la pompe (23) peut être implantée dans le patient.

13. Ensemble d'électrode d'implant cochléaire selon l'une quelconque des revendications précédentes, dans lequel le fluide bio-actif comprend un ou plusieurs facteurs neurotrophiques sélectionnés parmi le groupe comprenant GDNF, BDNF, NT-3, FGF et LIF.
